# EUROPEAN PATENT APPLICATION

(11) **EP 4 837 498 A2**
(43) Date of publication of application: **11.11.2026**
(21) Application number: 26174568.1
(22) Date of filing: 24.04.2026
(51) Int. Cl.: A61B 1/00, A61B 1/05, A61B 1/06, A61B 1/005

(54) **SHAFT, SURGICAL INSTRUMENT AND DISTAL END CAP**

(30) Priority: 09.05.2025 LU 601532
(71) Applicant: Karl Storz SE & Co. KG, 78532 Tuttlingen (DE)
(72) Inventor: TOMBERG, Johan, 76401 Laagri (EE)

(57) **Abstract**

The present invention provides a shaft (1) for a surgical instrument, in particular for a flexible endoscopic instrument, comprising: a tubular shaft structure (2) having an inner surface (2a) and an outer surface (2b) facing away from the inner surface (2a), wherein the shaft structure (2) is configured longitudinal with a distal end (3) and a proximal end (4); and an electrical conductor (5), which is disposed on the outer surface (2b) or integrated in a tube wall between the inner surface (2a) and the outer surface (2b) and extends between the distal end (3) and the proximal end (4). Further the present invention provides a surgical instrument (100) comprising such a shaft (1).

## Description

### TECHNICAL FIELD

The present invention pertains to a shaft for a surgical instrument, in particular for a flexible endoscopic instrument. Furthermore, the present invention pertains to a surgical instrument, in particular flexible endoscopic instrument, with such a shaft, and a distal end cap for a shaft of a surgical instrument.

### BACKGROUND

Surgical tube shaft instruments such as flexible endoscopic instruments are used for a variety of applications in medicine and technology. Such flexible endoscopic instruments comprise a flexible elongated shaft which is suitable for insertion into a cavity, such as an internal body cavity or a cavity of a technical object. As a rule, an endoscopic instrument lens is arranged at the tip of the endoscopic instrument shaft to generate an image of a scene in the observed cavity. To record and transmit the endoscopic image from the distal (i.e. far from the observer) to the proximal (i.e. close to the observer) end region of the endoscopic instrument, an ordered bundle of optical fibers running inside the shaft can be provided, for example, or an electronic image sensor, such as a CCD chip, which is arranged in the region of the distal end of the shaft and whose signals are transmitted to the proximal end region via electrical lines running inside the shaft. Since there is usually not enough light in the observed cavity, a light guide system can also be arranged inside the shaft to transport light to the distal end of the endoscopic instrument, where it is used to illuminate the cavity. Further, the endoscopic instrument shaft may include one or more working channels for passing endoscopic working instruments from the proximal to the distal end portion of the shaft for performing manipulations within the cavity.

Surgical tube shaft instruments are also known which comprise a flexible elongated shaft which is also suitable for insertion into a cavity, such as an internal body cavity or a cavity of a technical object. Such a flexible endoscopic instrument can be used to carry out manipulations in the cavity and for this purpose can be designed, for example, as a grasping instrument for grasping and manipulating tissue or objects in the cavity inside the body or in the cavity of a technical object. For this purpose, a tool is arranged at the distal end of the shaft, which can be operated from the proximal end of the shaft via a transmission means running inside the shaft. Such a flexible endoscopic instrument usually does not have its own optics for recording an endoscopic image, but can be used in particular together with a flexible endoscopic instrument.

It is often desirable to be able to angle the distal end of the shaft, i.e. the tip of the endoscope or endoscopic instrument, in order to facilitate the insertion of the endoscope or endoscopic instrument through a non-flexible endoscopic instrument through a non-rectilinear channel, to be able to move the tip within a cavity in a lateral direction and to be able to change the viewing direction of an optical system arranged in the endoscopic instrument tip or the working direction of a tool arranged at the tip of the endoscopic instrument. For this purpose, the shaft has a controllable section, in particular a controllable end section, which can be actively angled by a desired amount in a desired direction and can be controlled for this purpose from the proximal end of the endoscopic instrument. The shaft for a flexible endoscopic instrument need not itself have a tool and a transmission means, but can for example comprise a working channel into which a flexible endoscopic working instrument which cannot be actively angled and which has such a tool can be inserted up to the distal end of the shaft and possibly beyond, so that the flexible working instrument can be angled with the aid of the shaft.

The shaft is usually part of the endoscope, which transfers the torque and force from a handle to a distal section. Since the shaft is hollow it protects also the internal components, like the working channel, light/imager cables, springs and/or push-pull/steering wires.

In known endoscopes the imager cables are typically placed inside the endoscope shaft separately. They take up space inside the endoscope insertion portion, limiting a minimum size of the endoscope shaft. Due to this nature, the imager cables are usually very difficult to assemble and highly challenging when automated manufacturing is desired. Additionally, a mechanical durability of the endoscope can be compromised due to a possibility of component engagement and their small size.

### DISCLOSURE OF THE INVENTION

It is an object of the present invention to provide a shaft, which can simplify and miniaturize surgical instrument designs keeping in mind low cost and usability.

According to the invention, this problem is solved by a shaft for a surgical instrument with the features of claim 1 and/or by a surgical instrument with the features of claim 11 and/or by a distal end cap for a shaft of a surgical instrument with the features of claim 16.

According to a first aspect of the invention, a shaft for a surgical instrument, in particular for a flexible endoscopic instrument, is provided. The shaft comprises a tubular shaft structure having an inner surface and an outer surface. The outer surface faces away from the inner surface. The shaft structure is configured longitudinal with a distal end and a proximal end. Further, the shaft comprises an electrical conductor, which is disposed on the outer surface or integrated in a tube wall between the inner surface and the outer surface. Furthermore, the electrical conductor extends between the distal end and the proximal end.

According to a second aspect of the invention, a surgical instrument, in particular flexible endoscopic instrument, is provided. The surgical instrument comprises a shaft according to the first aspect of the invention and an optical image device for generating image data of an area in front of the distal end of the shaft. The optical image device is arranged at the distal end and electronically connected to the electrical conductor.

According to a third aspect of the invention, a distal end cap for a shaft of a surgical instrument is provided. The distal end cap comprises a contact zone for mounting the distal end cap to a distal end of a shaft structure of a shaft. Further, the distal end cap comprises at least one electrical connector that is configured to provide an integrated conductive pathway to electrically connect an optical image device, a light source and/or a temperature and/or pressure sensor device to an electrical conductor of the shaft structure.

A fundamental concept of the invention is to provide an endoscope working vertebrae or shaft, respectively, that is combined with the electrical conductor in order to transfer an image from the optical image device respectively camera unit.

The shaft structure in particular accommodates steering wires, a working channel and an optical image device.

The present invention can be applied to all flexible endoscopes that have an electronic camera head and a shaft. A distal end of the working channel corresponds to the distal end of the shaft or surgical instrument.

A particular advantage in the solution according to an aspect of the invention is that space can be saved in the shaft and much more simple assembly procedures can be provided.

A further advantage of the present invention is that an automation process can be optimized.

The shaft structure can be based on an extruded profile, for example. That means, the shaft structure can be manufactured by an extrusion process. The electrical conductor could be applied with the following manufacturing processes used to metallize plastics, for example: an arc/flame spraying, an electroless plating, a generative manufacturing like 3D-printing, an electroplating or a vacuum metalizing. It is still not very common for elastic/thin walled tube parts, so it could be a manufacturing challenge. Furthermore, the electrical conductor can be made with one of the methods: an injection molded plastic part with metallic inserts when injection molding, a metallic coating with the methods mentioned above, small metal ribbons placed before assembly, and molded interconnected device (MID)-technology. Benefits with this combination would remove any separate wiring from the shaft side, such that the assembly is much more compact and easier to assemble or automate.

If the surgical instrument is to be operated by a user such as a surgeon, the surgical instrument comprises a handle as a grip for the user. The operating interface then transmits a force or torque, which is applied by the user to the handle via a suitable actuation (e.g. movable handle leg), to a force transmission element for example.

If the surgical instrument is to be connected to a robot, the surgical instrument comprises a corresponding connection interface for the robot, which can apply an axial force or torque to the force transmission element via the operating interface.

Advantageous embodiments and further developments emerge from the description with reference to the figures.

According to some embodiments of the invention, the outer surface comprises a recess, whose shape corresponds to a cross sectional shape of the electrical conductor such that the electrical conductor is integrated in the recess.

According to some further embodiments of the invention, the electrical conductor is configured as a coating, in particular as a graphene coating, as a electro spark coating or as a physical vapor deposition coating. The graphene coating can be advantageously used for very small applications. Graphene is superconductive and has an extremely thin wall, hence the graphene coating does not have an impact on dimensions of the shaft.

According to some further embodiments of the invention, the shaft further comprises a cladding for insulating the electrical conductor against an environment, wherein the cladding is disposed on the outer surface of the shaft structure. The cladding can have a porous material that provides a flexibility, a kink resistance and good frictional properties for the shaft.

Optionally, the shaft structure and the cladding can have a press fit and/or be bonded together materially.

According to some further embodiments of the invention, the cladding omits an area of the electrical conductor, wherein the area is arranged at a region of the distal end. Alternatively or additionally, the cladding can omit an area of the electrical conductor, wherein the area is arranged at a region of the proximal end. Hence, at at least one end region of the shaft structure, a small area of the shaft structure respectively inner tube might not be coated by the cladding respectively insulating cover. This can provide a simple practical way for electrical connections.

For example, a semi-round flexprint device can be attached to a camera unit, a temperature and/or pressure sensor device and/or a light source and can be wrapped around the shaft structure providing a simple but rigid electrical connection between the camera unit, the a temperature and/or pressure sensor device and/or the light source and the electrical conductor. Thereby, the camera unit, the a temperature and/or pressure sensor device and/or the light source can be arranged at the distal end. Furthermore, the semi-round flexprint device can be in electrical contact with the electrical conductor. Optionally, the shaft structure respectively its tube wall can act as a support element and/or a positioning element for the camera unit and the light source. Thus, an assembly of the camera unit and the light source can be very compact. The light source may be configured as a LED, for example.

According to some further embodiments of the invention, the electrical conductor is coated with a dielectric insulator material. Hence, the shaft structure and the electrical conductor integrated in the recess of the shaft structure may form a single layer shaft. Such a single layer shaft can be insulated against an environment by the dielectric insulator material coating, nevertheless without an additional cladding.

In particular, the above described features can be combined with the electrical conductor disposed on the outer surface. The following features can be combined with the electrical conductor being either disposed on the outer surface or integrated in the tube wall between the inner surface and the outer surface.

According to some further embodiments of the invention, the electrical conductor is configured as a metal ribbon, which is oriented in a way that it does not affect deflection properties of the shaft. For example, the electrical conductor can comprise thin metal ribbons oriented in a way that they do not affect deflection properties of the shaft. The metal ribbon can be oriented straight, like a wave or in a spiral respectively helical form. The helical orientation of the metal ribbon can improve the mechanical properties of the shaft structure, in particular a stiffness and/or a kink resistance.

For example, the shaft structure can have helical metal ribbons as the electrical conductor. The electrical conductor can be arranged inside the shaft structure. That means, a cladding around the shaft structure is not needed since the electrical conductor is electrically insulated by the shaft structure, but nevertheless the shaft structure can be coated by a cladding. In some embodiments, the metal ribbons can use round wires, which are oriented linear and helical for the same purpose.

According to some further embodiments of the invention, the electrical conductor is configured as a carbon ribbon. The carbon ribbon can be a carbon fiber ribbon, a carbon line or the like.

According to some further embodiments of the invention, the electrical conductor is configured as a metal coil or braid. Hence, the metal coil or braid can be utilized not just for mechanical properties, but also for electrical properties. That means, in addition to a kink resistance and a torsion resistance the metal coil or braid can provide electrical purposes, wherein the shaft is kept flexible.

For example, the electrical conductor can be made of copper, carbon, graphene or another material with a similar or better conductivity.

According to some further embodiments of the invention, the inner surface is configured for communicating fluids or medical tools. That means, the tubular shaft structure corresponds to the working channel. In particular, the inner surface can have properties like the working channel. The tubular shaft structure or the inner surface can be made of a biocompatible material like polyurethane, thermoplastic elastomer or impregnated pellethane. Hence, the shaft structure can be used to transfer liquids and/or surgical tooling from the surgical instrument to inside the patient depending on the medical procedure.

The working channel can be inserted into the tubular shaft structure. The working channel can be mounted or guided at least at an end, in particular the proximal end, of a rod in the surgical instrument. In particular, the rod can be mounted or guided in the tubular shaft structure in such a way that the working channel cannot move or rotate axially relative to the shaft structure.

Optionally, the shaft structure, the electrical conductor, the working channel and a steerable section of the shaft may be a single component.

According to some further embodiments of the invention, the surgical instrument further comprises a light source, which is electronically connected to the electrical conductor.

According to some further embodiments of the invention, the shaft is configured as a flexible shaft for being articulatable at least in one plain.

According to some further embodiments of the invention, the surgical instrument further comprises an operating interface, which is arranged at the proximal end of the shaft and configured to operate the shaft.

For example, the surgical instrument can comprise a tool inlet adapter. The shaft structure can be connected to the tool inlet adapter, which is electronically coupled to an electronic circuit, for example a printed circuit board. Thereby, the tool inlet adapter can contact the shaft structure at its proximal end or near the proximal end. Hence, data and/or electrical energy can be transferred by the tool inlet adapter between the shaft structure and the electronic circuit.

According to some further embodiments of the invention, the surgical instrument further comprises two steering wires extending from the operating interface through the flexible shaft for articulating the flexible shaft.

The operating interface and/or the surgical instrument can be made of a plastic material. For example, the surgical instrument can be completely made of a plastic material, in particular when the surgical instrument is intended to use only once.

For example, the distal end cap can be made of plastic. Furthermore, the at least one electrical connector can be configured as a molded in part or as a metallic coating. In some embodiments, the shaft can comprise the shaft structure and the distal end cap. For example, the distal end cap can be glued on the distal end of the shaft structure. In particular, the distal end cap is mounted on the shaft structure such that the at least one electrical connector is in contact with the electrical conductor. The at least one electrical connector can be configured as cold plated copper ribbon.

The above embodiments and further developments can be combined with one another arbitrarily, as far as appropriate. Further possible configurations, developments and implementations of the invention are also combinations of features of the invention described above or below for the exemplary embodiments that are not explicitly cited. In particular, a person skilled in the art will also add individual aspects as improvements or additions to the respective basic form of the present invention.

### BRIEF DESCRIPTION OF THE DRAWINGS

The present invention is explained more specifically below on the basis of the exemplary embodiments indicated in the schematic figures, in which:
Fig. 1 shows a handheld surgical instrument, in particular a steerable flexible endoscopic instrument, in an overall view according to an embodiment of the invention;
Fig. 2 shows an electrical connection interface of the shaft structure in the operating interface of the handheld surgical instrument of Fig. 1;
Fig. 3 shows a perspective view of a shaft for a surgical instrument according to a further embodiment of the invention;
Fig. 4 shows a perspective view of the shaft of Fig. 3, wherein a semi-round flexprint device, a camera unit and a light source are attached to a distal end of the shaft;
Fig. 5 shows a perspective view of a shaft for a surgical instrument according to a further embodiment of the invention;
Fig. 6 shows a distal end cap according to a further **em**bodiment of the invention for the shaft of Fig. 5;
Fig. 7 shows the distal end cap of Fig. 6 mounted on the shaft structure of Fig. 5;
Fig. 8 shows a perspective view of a shaft for a surgical instrument according to a further embodiment of the invention; and
Fig. 9 shows a perspective view of a shaft for a surgical instrument according to a further embodiment of the invention.

The accompanying figures are intended to convey a further understanding of the embodiments of the invention. They illustrate embodiments and are used in conjunction with the description to explain principles and concepts of the invention. Other embodiments and many of the cited advantages emerge in light of the drawings. The elements of the drawings are not necessarily shown to scale in relation to one another. Direction-indicating terminology such as for example "at the top", "at the bottom", "on the left", "on the right", "above", "below", "horizontally", "vertically", "at the front", "at the rear" and similar statements are merely used for explanatory purposes and do not serve to restrict the generality to specific configurations as shown in the figures.

In the figures of the drawing, elements, features and components that are the same, have the same function and have the same effect are each provided with the same reference signs - unless explained otherwise.

### DETAILED DESCRIPTION OF DRAWINGS

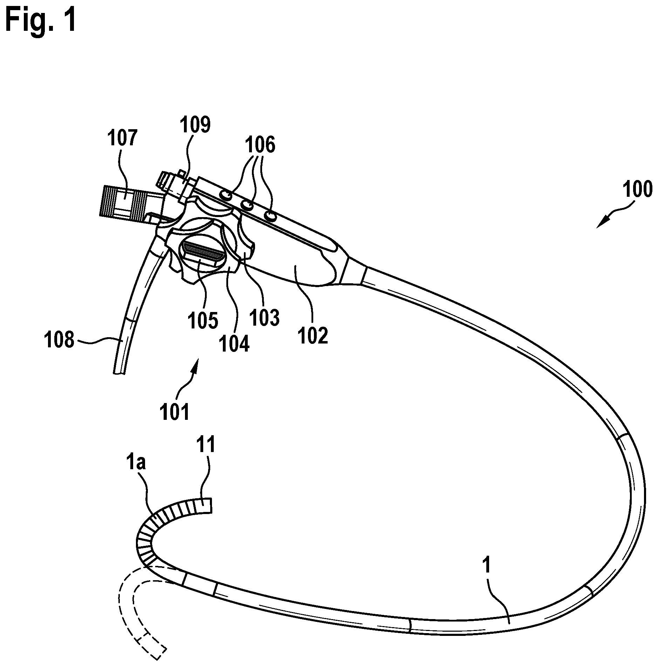

Fig. 1 shows an embodiment of a handheld surgical instrument 100, in particular of a steerable flexible endoscopic instrument 100, in an overall view.

As shown schematically in Fig. 1, the flexible endoscopic instrument 100 typically comprises a handpiece 101 and a shaft 1, the handpiece 101 being attached to a proximal end of the shaft 1. The handpiece 101 can have an outer housing 102 made of a plastic and/or metallic material. On a lower side of the housing 102 a first hand wheel 103 and a second hand wheel 104 are arranged for controlling a deflection of a steerable section 1a of the shaft 1, as is described below. Typically the first and the second hand wheel 103, 104 are arranged coaxially, and at an exterior side of the second hand wheel 104 a knob 105 for controlling a deflection brake relating to the second hand wheel 104 is provided. Further, the handpiece 101 may exhibit a multiplicity of control buttons 106 for controlling various functions of the flexible endoscopic instrument 100, such as for controlling the imaging and/or illumination system and/or irrigation and suction pumps, for example. The handpiece 101 may be connectable to an external video unit or a video monitor via connector 107 and to an external light source via light cable 111. Moreover, an instrument port 112 may be provided for inserting endoscopic instruments to be advanced through one or more respective channels to a distal end of the shaft 1 for manipulating tissue or other objects within a cavity into which the shaft 1 can be inserted.

At its distal end the shaft 1 comprises a steerable section 1a. The steerable section 1a may form a distal end section of the shaft 1 or, as shown in Fig. 1, may carry a distal end cap 11, which may accommodate an imaging optics and an electronic image sensor for providing an endoscopic image of a cavity into which the shaft 1 is inserted. The image signal generated by the image sensor may be transmitted via electric cables extending through the shaft 1 and the handpiece 101 to the connector 107 for being processed and displayed by an external video unit. The shaft 1 in total is flexible to an extent to be advanced through an endoscopic access or a hollow organ towards a cavity to be observed, being capable of adapting to a curved shape of the access or the organ. The steerable section 1a, on the other hand, is capable of being flexed actively by turning the hand wheels 103, 104. To this end the steerable section 1a comprises an inner structure of a plurality of shaft sections, thus being deflectable or articulatable in one or more planes. The plurality of shaft sections can be covered by a flexible tube to form a smooth outer surface, the shaft 1 in total having a uniform cross-sectional shape and diameter.

For controlling the deflection of the plurality of shaft sections two counteracting control wires (not shown in Fig. 1) are provided extending on opposite sides within the plurality of shaft sections, by a longitudinal movement of which the steerable section 1a can be bent to one or the other side, as indicated symbolically in Fig. 1. The control wires extend along the shaft 1 and are connected at their proximal ends to a deflection control mechanism arranged within the handpiece 101, which can be operated by a user by turning the hand wheels 103, 104 for deflecting the steerable section 1a. In the exemplary embodiment shown, the steerable section can be bent or articulated within a first plane that corresponds to the plane of the drawing, a deflection angle being controllable by rotating the first hand wheel 103. Further, the steerable section 1a can be bent in a second plane perpendicular to the first plane and perpendicular to the plane of the drawing, a corresponding deflection angle being controllable by rotating the second hand wheel 104.

The shaft 1 exemplarily comprises a tubular shaft structure 2 and an electrical conductor 5. The tubular shaft structure 2 has an inner surface 2a and an outer surface 2b facing away from the inner surface 2a. The shaft structure 2 is configured longitudinal with a distal end 3 and a proximal end 4. The electrical conductor 5 is disposed on the outer surface 2b or integrated in a tube wall between the inner surface 2a and the outer surface 2b and extends between the distal end 3 and the proximal end 4. For example, the electrical conductor 5 can extend from a region of the distal end 3 to a region of the proximal end 4.

Optionally, the surgical instrument 100 can comprise an optical image device for generating image data of an area in front of the distal end 3 of the shaft 1, wherein the optical image device is arranged at the distal end 3 and electronically connected to the electrical conductor. Furthermore, the surgical instrument 100 can comprise a light source 110, which is electronically connected to the electrical conductor, and two steering wires extending from the operating interface 102 through the shaft 1 for articulating the shaft.

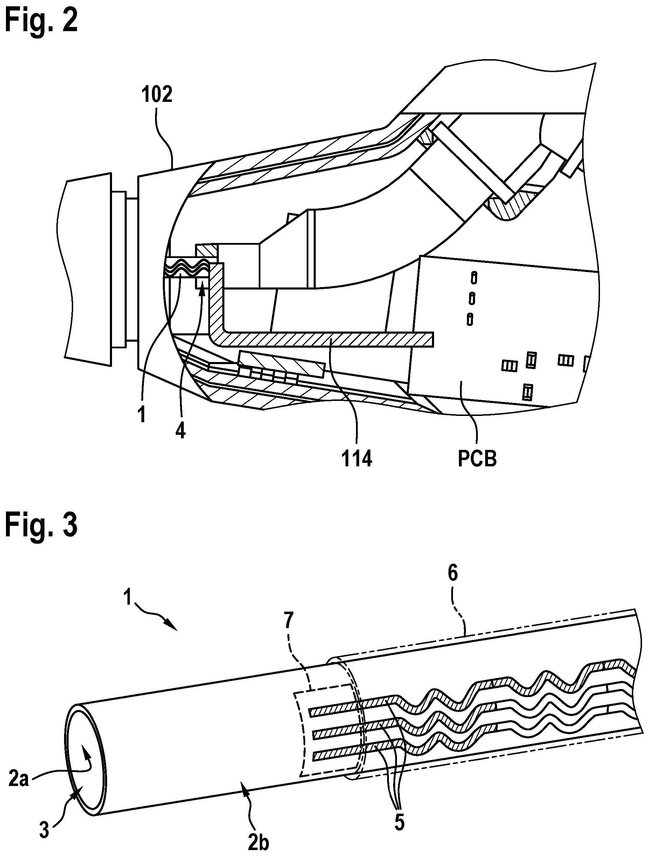

Fig. 2 shows an electrical connection interface of the shaft structure 2 in the operating interface 102 of the handheld surgical instrument 100 of Fig. 1.

The shaft structure 2 can be connected to a tool inlet adapter 114, which is electronically coupled to an electronic circuit, for example a printed circuit board PCB. Thereby, the tool inlet adapter 114 can contact the shaft structure 2 at its proximal end 4 or near the proximal end 4. Hence, data and/or electrical energy can be transferred by the tool inlet adapter 114 between the shaft structure 2 and the electronic circuit PCB. Thus, there is no need to create a separate electrical connection for the shaft structure 2.

Fig. 3 shows a perspective view of a shaft 1 for a surgical instrument 100 according to a further embodiment of the invention.

The shaft 1 comprises a tubular shaft structure 2, an electrical conductor 5 and an optional cladding 6. The tubular shaft structure 2 has an inner surface 2a and an outer surface 2b facing away from the inner surface 2a. The shaft structure 2 is configured longitudinal with a distal end 3 and a proximal end 4.

Here, the electrical conductor 5 is disposed on the outer surface 2b and extends between the distal end 3 and the proximal end 4. For example, the electrical conductor 5 can extend from a region of the distal end 3 to a region of the proximal end 4. In particular, the outer surface 2b comprises a recess, whose shape corresponds to a cross sectional shape of the electrical conductor 5 such that the electrical conductor 5 is integrated in the recess.

The cladding 6 can be configured for insulating the electrical conductor 5 against an environment. For example, the cladding 6 is disposed on the outer surface 2b of the shaft structure 2. The cladding 6 can have a porous material that provides a flexibility, a kink resistance and good frictional properties for the shaft. As it is exemplarily illustrated in Fig. 3, the cladding 6 can omit an area 7 of the electrical conductor 5, wherein the area 7 is arranged at a region of the distal end 3. Alternatively or additionally, the cladding 6 can omit an area 7 of the electrical conductor 5, wherein the area 7 is arranged at a region of the proximal end 4. Hence, the area 7 of the shaft structure 2 respectively inner tube might not be coated by the cladding 6 respectively insulating cover in the end region of the distal end 3.

Optionally, the electrical conductor 5 can be coated with a dielectric insulator material. Hence, the shaft structure 2 and the electrical conductor 5 integrated in the recess of the shaft structure 2 may form a single layer shaft. Such a single layer shaft can be insulated against an environment by the dielectric insulator material coating, nevertheless without an additional cladding 6.

The electrical conductor 5 is exemplarily configured as a metal ribbon, which is oriented in a way that it does not affect deflection properties of the shaft 1. In particular, the electrical conductor 5 can comprise a plurality of thin metal ribbons oriented in a way that they do not affect deflection properties of the shaft, as it is illustrated in Fig. 3.

But the electrical conductor 5 is not limited to the illustrated metal ribbon and can also be configured as a carbon ribbon. The carbon ribbon can be a carbon fiber ribbon, a carbon line or the like. Alternatively or additionally, the electrical conductor 5 can be configured as a metal coil or braid.

For example, the electrical conductor 5 can be made of copper, carbon, graphene or another material with a similar or better conductivity.

Further, the inner surface 2a can be configured for communicating fluids or medical tools. That means, the tubular shaft structure 2 corresponds to the working channel. In particular, the inner surface 2a can have properties like the working channel. The shaft structure 2 or the inner surface 2a can be made of a biocompatible material like polyurethane, thermoplastic elastomer or impregnated pellethane.

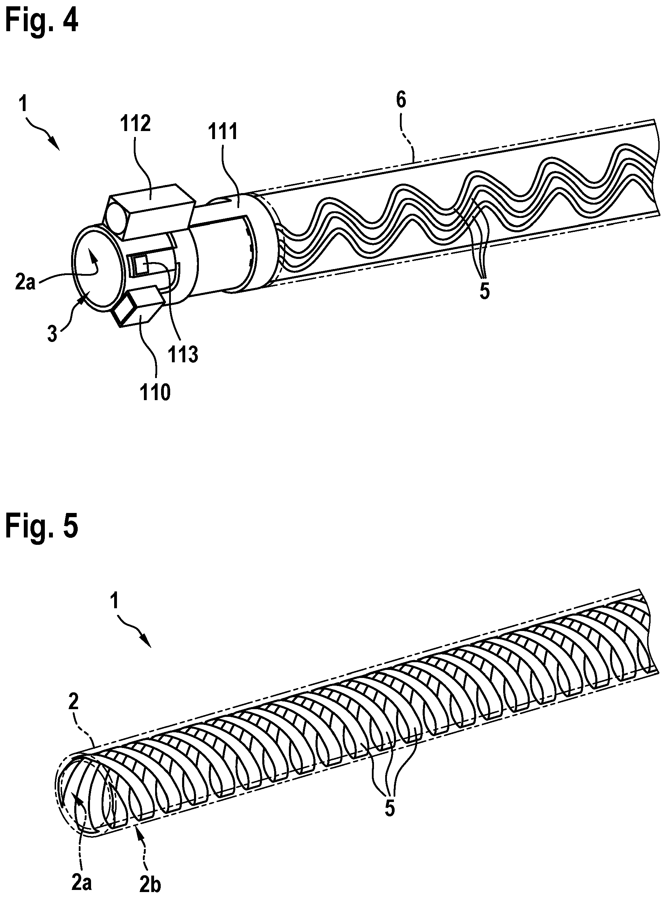

Fig. 4 shows a perspective view of the shaft 1 of Fig. 3, wherein a semi-round flexprint device 111, a camera unit 112, a temperature and/or pressure sensor device 113 and a light source 110 are attached to a distal end 3 of the shaft 1.

For example, the semi-round flexprint device 111 can be attached to the camera unit 112, the temperature and/or pressure sensor device 113 and the light source 110. Furthermore, the semi-round flexprint device 111 can be wrapped around the shaft structure 2 providing a simple but rigid electrical connection between the camera unit 112 and the temperature and/or pressure sensor device 113 and the light source 110 and the electrical conductor 5. Thereby, the camera unit 112, the temperature and/or pressure sensor device 113 and the light source 110 can be arranged at the distal end 3. Furthermore, the semi-round flexprint device 111 can be in electrical contact with the electrical conductor 5.

Optionally, the shaft structure 2 respectively its tube wall can act as a support element and/or a positioning element for the camera unit 112 and the light source 110. The light source 110 may be configured as a LED, for example.

Fig. 5 shows a perspective view of a shaft 1 for a surgical instrument according to a further embodiment of the invention.

The shaft of Fig. 5 substantially comprises the same features as the shaft 1 of Figs. 3 and 4 unless described otherwise. Here, the electrical conductor 5 is exemplarily configured as a coating, in particular as a graphene coating, as an electro spark coating or as a physical vapor deposition coating.

The electrical conductor 5 can be formed as a spiral respectively helix around the shaft structure 2. For example, the shaft 1 can have helical metal ribbons as the electrical conductor 5. In some embodiments, the metal ribbons can use round wires, which are oriented linear and helical for the same purpose.

In Fig. 5, the electrical conductor 5 comprises four metal ribbons oriented helically around the shaft structure 2. In particular, the four metal ribbons 5 are integrated in between the inner surface 2a and the outer surface 2b.

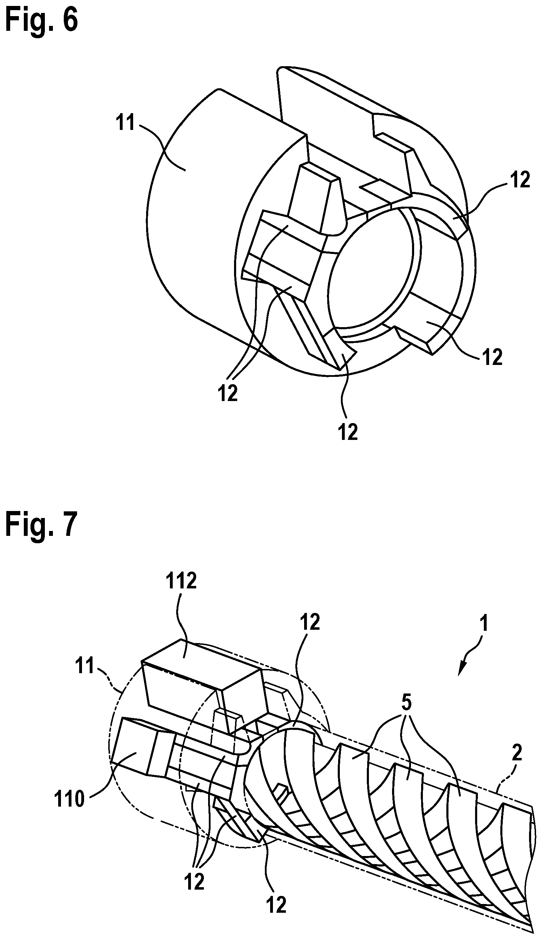

Fig. 6 shows a distal end cap 11 according to a further embodiment of the invention for the shaft 1 of Fig. 5. Fig. 7 shows the distal end cap 11 of Fig. 6 mounted on the shaft structure 2 of Fig. 5.

The distal end cap 11 comprises at least one, here four, integrated electrical connector 12. For example, the distal end cap 11 is made of plastic. The at least one electrical connector 12 is configured to provide an integrated conductive pathway in order to electrically connect the optical image device 112, the light source 110 and the temperature and/or pressure sensor device 113 to the electrical conductor 5 of the shaft structure 2, wherein each can be attached to or housed in the distal end cap 11. Furthermore, the at least one electrical connector 12 can be configured as a molded in part or as a metallic coating.

As it is illustrated in Fig. 7, the shaft 1 can comprise the shaft structure 2 and the distal end cap 11. For example, the distal end cap 11 is glued on the distal end 3 of the shaft structure 2. In particular, the distal end cap 11 is mounted on the shaft structure 2 such that the four electrical connectors 12 are in contact with the four metal ribbons **5.** Two of the four electrical connectors 12 are coupled to the light source 110 and the other two of the four electrical connectors 12 are coupled to the optical image device 112. Both the light source 110 and the optical image device 112 are mounted in the distal end cap 11. The electrical connectors 12 can be configured as cold plated copper ribbons.

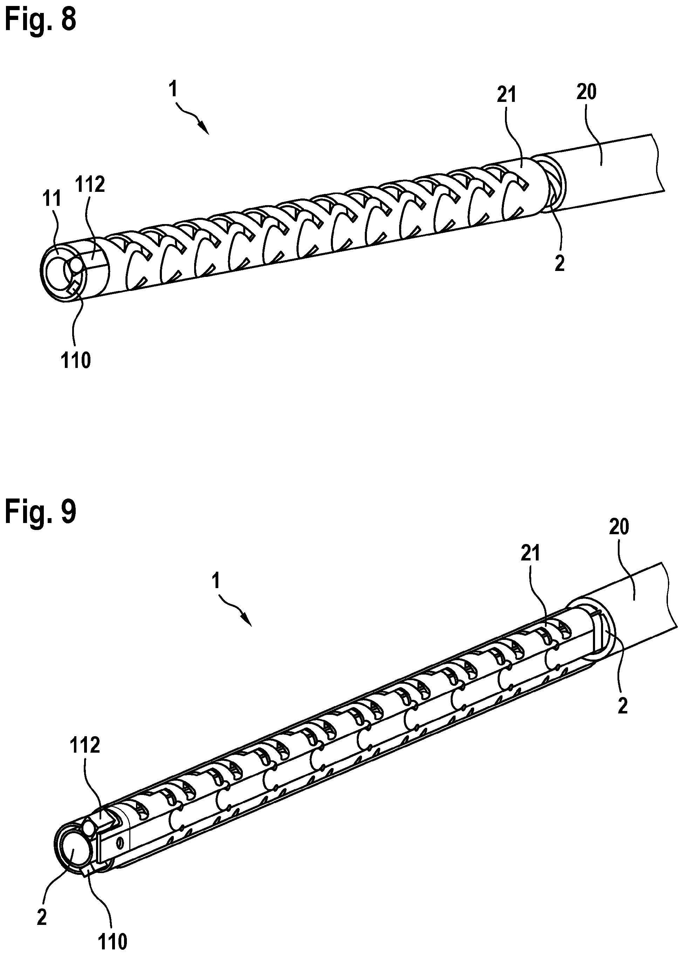

Fig. 8 shows a perspective view of a shaft 1 for a surgical instrument according to a further embodiment of the invention.

Here, the shaft 1 exemplarily comprises the tubular shaft structure 2 and the electrical conductor 5 according to one of the embodiments in Figs. **3-7****.** The tubular shaft structure 2 and the electrical conductor 5 might form an inner layer respectively inner structure of the shaft 1. Additionally, the shaft 1 can comprise an outer layer 20.

The outer layer 20 can comprise a controllable respectively movable section 21, which is arranged in a steerable section of the shaft 1. The optical image device 112 and the light source 110 might be mounted in a distal end cap 11 of Figs. 6 and **7****.** The outer layer 20 can also be named an insertion shaft. The insertion shaft might be the portion of a surgical instrument 100, in particular of a flexible endoscope, that is inserted inside the patient, wherein the controllable section 21 helps to navigate inside the patient.

The controllable section 21 radially surrounds the shaft structure 2. Moreover, the outer layer 20 radially surrounds the shaft structure 2. Thus, both the controllable section 21 and the outer layer 20 radially surround the shaft structure 2, wherein the distal end 3 is not covered by them. The inner layer is housed inside the insertion shaft and interconnects the handpiece and the distal end openings.

The outer layer 20 can be configured as a coating, for example like a UV reactive flexible polymer coating. In this way, the manufacturing process like spraying can apply the coating and it will be UV hardened afterwards. For example, the outer layer 20 can be configured as a multilumen shaft with cavities for control wires, which are used for moving the controllable portion 21.

The controllable section 21 comprises cutouts that are configured and arranged such that the controllable section 21 is articulatable in at least one plane.

Fig. 9 shows a perspective view of a shaft 1 for a surgical instrument according to a further embodiment of the invention.

The shaft 1 of Fig. 9 substantially comprises the same features as the shaft 1 of Fig. 8 unless described otherwise. Here, the controllable section 21 comprises a recess for the light source 110 and a recess for the optical image device 112. Furthermore, the controllable section 21 sectionwise surrounds the distal end 3 of the shaft structure 2.

Moreover, here the controllable section 21 comprises different cutouts than in Fig. 8 but for the same purpose of articulating the controllable section 21.

In the detailed description above, various features have been combined in one or more examples in order to improve the rigorousness of the illustration. However, it should be clear in this case that the above description is of merely illustrative but in no way restrictive nature. It serves to cover all alternatives, modifications and equivalents of the various features and exemplary embodiments. Many other examples will be immediately and directly clear to a person skilled in the art on the basis of his knowledge in the art in consideration of the above description.

The exemplary embodiments have been chosen and described in order to be able to present the principles underlying the invention and their application possibilities in practice in the best possible way. As a result, those skilled in the art can optimally modify and utilize the invention and its various exemplary embodiments with regard to the intended purpose of use. In the claims and the description, the terms "including" and "having" are used as neutral linguistic concepts for the corresponding terms "comprising". Furthermore, use of the terms "a", "an" and "one" shall not in principle exclude the plurality of features and components described in this way.

While at least one exemplary embodiment of the present invention(s) is disclosed herein, it should be understood that modifications, substitutions and alternatives may be apparent to one of ordinary skill in the art and can be made without departing from the scope of this disclosure. This disclosure is intended to cover any adaptations or variations of the exemplary embodiment(s). In addition, in this disclosure, the terms "comprise" or "comprising" do not exclude other elements or steps, the terms "a" or "one" do not exclude a plural number, and the term "or" means either or both. Furthermore, characteristics or steps which have been described may also be used in combination with other characteristics or steps and in any order unless the disclosure or context suggests otherwise. This disclosure hereby incorporates by reference the complete disclosure of any patent or application from which it claims benefit or priority.

### REFERENCE LIST

- 1: shaft
- 1a: steerable section
- 2: tubular shaft structure
- 2a: inner surface
- 2b: outer surface
- 3: distal end
- 4: proximal end
- 5: electrical conductor
- 6: cladding
- 7: area
- 11: distal end cap
- 20: outer layer
- 21: controllable section
- 100: surgical instrument
- 101: handpiece
- 102: operating interface
- 103: first hand wheel
- 104: tool or accessory
- 105: handle
- 106: movable handle limb
- 107: accessory interface
- 108: light cable
- 109: instrument port
- 110: light source
- 111: semi-round flexprint device
- 112: optical image device/camera unit
- 113: temperature and/or pressure sensor device
- 114: tool inlet adapter

- PCB: printed circuit board

## Claims

1. Shaft (1) for a surgical instrument (100), in particular for a flexible endoscopic instrument, comprising:
a tubular shaft structure (2) having an inner surface (2a) and an outer surface (2b) facing away from the inner surface (2a), wherein the shaft structure (2) is configured longitudinal with a distal end (3) and a proximal end (4); and
an electrical conductor (5), which is disposed on the outer surface (2b) or integrated in a tube wall between the inner surface (2a) and the outer surface (2b) and extends between the distal end (3) and the proximal end (4).

2. Shaft (1) according to claim 1,
**characterized in that**
the outer surface (2b) comprises a recess, whose shape corresponds to a cross sectional shape of the electrical conductor (5) such that the electrical conductor (5) is integrated in the recess.

3. Shaft (1) according to claim 1 or 2,
**characterized in that**
the electrical conductor (5) is configured as a coating, in particular as a graphene coating, as a electro spark coating or as a physical vapor deposition coating.

4. Shaft (1) according to one of the preceding claims, further comprising
a cladding (6) for insulating the electrical conductor (5) against an environment, wherein the cladding (6) is disposed on the outer surface (2b) of the shaft structure (2).

5. Shaft (1) according to claim 4,
**characterized in that**
the cladding (6) omits an area (7) of the electrical conductor (5), wherein the area (7) is arranged at a region of the distal end (3).

6. Shaft (1) according to one of the preceding claims, **characterized in that**
the electrical conductor (5) is coated with a dielectric insulator material.

7. Shaft (1) according to one of the preceding claims, **characterized in that**
the electrical conductor (5) is configured as a metal ribbon, which is oriented in a way that it does not affect deflection properties of the shaft (1).

8. Shaft (1) according to one of the preceding claims, **characterized in that**
the electrical conductor (5) is configured as a carbon ribbon.

9. Shaft (1) according to one of the preceding claims, **characterized in that**
the electrical conductor (5) is configured as a metal coil or braid.

10. Shaft (1) according to one of the preceding claims, **characterized in that**
the inner surface (2a) is configured for communicating fluids or medical tools.

11. Surgical instrument (100), in particular flexible endoscopic instrument, comprising:
a shaft (1) according to one of the preceding claims; and
an optical image device (112) for generating image data of an area in front of the distal end (3) of the shaft (1), wherein the optical image device (112) is arranged at the distal end (3) and electronically connected to the electrical conductor (5).

12. Surgical instrument (100) according to claim 11, further comprising
a light source (110), which is electronically connected to the electrical conductor (5).

13. Surgical instrument (100) according to claim 11 or 12, **characterized in that**
the shaft (1) is configured as a flexible shaft for being articulatable at least in one plain.

14. Surgical instrument (100) according to one of the claims 11 to 13,
further comprising
an operating interface (102), which is arranged at the proximal end (4) of the shaft (1) and configured to operate the shaft (1).

15. Surgical instrument (100) according to claim 13 and 14, further comprising
two steering wires extending from the operating interface (102) through the flexible shaft (1) for articulating the flexible shaft.

16. Distal end cap (11) for a shaft (1) of a surgical instrument (100), comprising:
a contact zone for mounting the distal end cap (11) to a distal end (3) of a shaft structure (2) of a shaft (1); and
at least one electrical connector (12) that is configured to provide an integrated conductive pathway to electrically connect an optical image device (112), a light source (110) and/or a temperature and/or pressure sensor device (113) to an electrical conductor (5) of the shaft structure (2)
